# NEUE EUROPÄISCHE PATENTSCHRIFT

(11) **EP 1 135 177 B2**
(45) Veröffentlichungstag und Bekanntmachung des Hinweises auf die Entscheidung über den Einspruch: **12.12.2007**
(45) Hinweis auf die Patenterteilung: 04.06.2003
(21) Anmeldenummer: 99963373.8
(22) Anmeldetag: 03.12.1999
(51) Int. Cl.: A61L 27/26, A61L 27/34, A61L 27/38, C08L 89/06, C08L 5/08

(54) **PORÖSE KOMPOSITMATRIX, DEREN HERSTELLUNG UND VERWENDUNG**
POROUS COMPOSITE MATRIX AND THE PRODUCTION AND USE THEREOF
MATRICE COMPOSITE POREUSE, SA PRODUCTION ET SON UTILISATION

(30) Priorität: 03.12.1998 DE 19855890
(43) Veröffentlichungstag der Anmeldung: 26.09.2001
(73) Patentinhaber: Nerlich, Michael, 93080 Pentling (DE)
(72) Erfinder: ANGELE, Peter, D-93053 Regensburg (DE); KUJAT, Richard, D-93186 Pettendorf (DE)
(74) Vertreter: Keller, Günter
(86) Internationale Anmeldenummer: PCT/EP1999/009444
(87) Internationale Veröffentlichungsnummer: WO 2000/032251

(56) Entgegenhaltungen:
- EP-A- 0 648 480
- WO-A-97/18842
- WO-A-97/45532
- WO-A-98/31345

## Beschreibung

Die Erfindung betrifft eine poröse Kompositmatrix aus einem Hyaluronsäurederivat und hydrolysiertem Kollagen, die als biokompatible und biodegradable Kompositmatrix Verwendung zur Reparatur muskuloskeletaler Defekte findet.

Zur Regeneration von Gewebedefekten ist die Verwendung einer körperverträglichen, langsam biodegradablen Matrix erforderlich, die unter geeigneten Bedingungen die Differenzierung eingebrachter Zellen mit ausgeprägter Produktion einer spezifischen interzellulären Matrix ermöglicht. Im Stand der Technik sind verschiedene Matrizes dieser Art bekannt.

Die WO 97/28192 offenbart ein Verfahren zur Herstellung prionenfreier Kollagenprodukte, die als schwammartiges Implantat verwendet werden können. Für okulare Anwendungen kann das Kollagenprodukt zur Erhöhung der Transparenz mit 5 Gew.-% Hyaluronsäure versetzt werden. Die Hyaluronsäure stimuliert außerdem die Zellinfiltration in das Implantat.

Die WO 91/18558 und WO 91/16867 sowie das US 4,880,429 offenbaren Kompositmatrizes aus Kollagen und bis zu 25 Gew.-% Glykosaminoglykanen wie beispielsweise Hyaluronsäure.

Die EP-A-0 784 985 offenbart einen porösen Kompositkörper, der ein bioabsorbierbares hydrophiles Material ausgewählt aus Gelatine, Hyaluronsäure und einem Hyaluronsäurederivat umfaßt. Der Körper wird zur Vermeidung einer verfrühten Resorption zusätzlich mit einer verzögert resorbierbaren Polymerschicht versehen.

Die WO 97/14376 offenbart eine Knochentransplantatmatrix aus Kollagen, die als Bindemittel Hyaluronsäure enthalten kann.

Das US 5,676,964 offenbart inter- und intramolekular vernetzte Ester von sauren Polysacchariden wie bevorzugt Hyaluronsäure. Diese Ester können als biodegradable, beispielsweise schwammige Materialien als chirurgische Gegenstände eingesetzt werden.

Die bekannten Matrizes zeigen jedoch deutliche Einschränkungen in der Bereitstellung von für die Differenzierung eingebrachter Zellen geeigneter Milieubedingungen (z.B. frühzeitige Resorption, nicht geeignete Matrixzusammensetzung) und sind auch im Hinblick auf die für die Handhabung notwendige Stabilität unbefriedigend.

Eine Aufgabe der vorliegenden Erfindung besteht somit darin eine Matrix zur Verfügung zu stellen, die die Zelldifferenzierung und interzellulare Matrixproduktion unterstützt und dann selbst langsam degradiert wird. Außerdem soll die Matrix eine ausreichende Stabilität aufweiten, die die Matrix nicht nur für eine Vorkultivierung von Zellen in vitro sondern auch für eine Implantation gut geeignet und leicht handhabbar macht.

Es wurde nun gefunden, daß diese Aufgabe durch eine Kompositmatrix aus einem Hyaluronsäurederivat und hydrolysiertem Kollagen bestimmter Zusammensetzung gelöst wird.

Die vorliegende Erfindung betrifft somit eine poröse Kompositmatrix, wobei die Matrix aus Matrixbildnern umfassend ein Hyaluronsäurederivat und hydrolysiertes Kollagen aufgebaut ist, und die Matrixbildner in einem Gewichtsverhältnisbereich von Hyaluronsäurederivat zu hydrolysiertem Kollagen von 30:70 bis 99:1 vorliegen.

Die erfindungsgemäße Kompositmatrix umfaßt als Matrixbildner ein Hyaluronsäurederivat und hydrolysiertes Kollagen bevorzugt in einem Gewichtsverhältnis von 60:40 bis 99:1 und besonders bevorzugt von etwa 70:30.

Bevorzugt ist die Matrix nur aus dem Hyaluronsäurederivat und dem hydrolysierten Kollagen aufgebaut.

Es hat sich gezeigt, daß Matrizes mit einem Hyaluronsäurederivatanteil von unter 30 Gew.-% technisch aufgrund einer zu geringen Stabilität nachteilig sind. umgekehrt konnte durch die erfindungsgemäße Kompositmatrix aus einem Hyaluronsäurederivat und hydrolysiertem Kollagen die Zelladhäsion, die Matrixbeladung mit Zellen und die darauffolgende Zelldifferenzierung gegenüber Matrizes aus 100% Hyaluronsäurederivat, wie sie beispielsweise aus dem eingangs genannten US 5,676,964 bekannt sind, deutlich verbessert werden.

Außerdem wird durch das Hyaluronsäurederivat eine verzögert abbaubare Komponente direkt in die Kompositmatrix aufgenommen. Hierdurch kann beispielsweise die aus der EP-A-0 784 985 bekannte zusätzliche Beschichtung vermieden werden oder eine sonst gegebenenfalls notwendige Kollagenderivatisierung, die aufgrund der zum Teil nachgewiesenen Toxizität der Derivatisierungsagentien unerwünscht ist.

Als hydrolysiertes Kollagen eignet sich partiell und/oder vollständig hydrolysiertes Kollagen, insbesondere Gelatine, d.h. Kollagen in stark hydrolysierter Form. Beispielsweise kann Gelatine vom Schwein oder vom Rind eingesetzt werden. Es können jedoch auch Gelatineformen mit einer höheren Aggregationsrate in Richtung Fibrillen eingesetzt werden. Stärker aggregiertes Kollagen kann zu einer Verbesserung der Matrixstabilität führen. Solche Kollagene mit unterschiedlich großen Degradationsformen können durch eine kontrollierte, langsame Hydrolyse von fibrillärem Kollagen erzeugt werden.

Das hydrolysierte Kollagen kann gewünschtenfalls zusätzlich derivatisiert und/oder quervernetzt sein.

Als Hyaluronsäurederivat zur Herstellung der erfindungsgemäßen Kompositmatrix werden Hyaluronsäureester wie Ethyl- und insbesondere Benzylester aufgrund seiner besseren biomechanischen Eigenschaften bevorzugt, wobei die Hyaluronsäure unterschiedliche Veresterungsgrade aufweisen kann. Beispiele für erfindungsgemäß einsetzbare Hyaluronsäureester sind in dem US-Patent Nr. 5,676,964 genannt.

Vorteilhaft ist das eingesetzte Hyaluronsäurederivat überwiegend hydrophob.

Ein bevorzugter Hyaluronsäureester ist ein Benzylester der Hyaluronsäure (HYAFF), der beispielsweise von der Firma "Fidia Advanced Biopolymers" aus Abano Therme in Italien bezogen werden kann. HYAFF wird in verschiedenen Veresterungsgraden angeboten, von denen erfindungsgemäß ein hochveresterter Hyaluronsäurebenzylester "HYAFF 11" (100% Benzylester), der als bereits zugelassenes Wundverbandmaterial "JALOSKIN" im Handel erhältlich ist, bevorzugt wird.

Andere Hyaluronsäureester mit niedrigeren Veresterungsgraden (beispielsweise HYAFF 11 p 75, ein Hyaluronsäurebenzylester mit einem Veresterungsgrad von ca. 75%) und/oder anderen Alkoholresten, wie beispielsweise Hyaluronsäureethylester (wie etwa HYAFF 7), oder mit Mischungen verschiedener Alkoholreste sind jedoch auch einsetzbar.

Die erfindungsgemäße Kompositmatrix ist porös, insbesondere offenporig. Bevorzugt haben die Poren in der Kompositmatrix einen durchschnittlichen Durchmesser im Bereich von 10-1000 µm, insbesondere 50-500 µm. Es hat sich gezeigt, daß zu große Poren (> 1000 µm) bei der Besiedelung mit Zellen zu einem hohen Zellverlust aus der Matrix, besonders bei kleinen Schwammdurchmessern führen. Bei zu kleinen Poren (< 100 µm) zeigt sich ein starker Siebeffekt und die Zellen können nicht in tieferen Matrixbereichen angesiedelt werden. Jedoch kann durch einen bestimmten Anteil kleinerer Poren eine niedrigere Dichte und eine lockerere Struktur der Kompositmatrix erreicht werden. Hierdurch kann eine Beschleunigung des Abbaus in vivo ohne Änderung der für die Zellen zugänglichen Porengröße erreicht werden.

Poren mit einem durchschnittlichen Durchmesser im Bereich von 100-350 µm und Poren mit einem durchschnittlichen Durchmesser im Bereich von 350-1000 µm haben sich als vorteilhaft erwiesen. Wenn eine niedrigere Dichte oder eine lockerere Struktur der Kompositmatrix erwünscht ist, können zusätzlich Poren im Bereich von 10-100 µm, insbesondere im Bereich von etwa 50 µm vorhanden sein. Auch können Poren in etwa gleicher Größe oder Poren mit einem Größegradienten bereitgestellt werden.

Zusätzlich kann die erfindungsgemäße Kompositmatrix chemisch oder physikalisch quervernetzt sein. Hierdurch läßt sich die biologische Abbaubarkeit der Kompositmatrix je nach Bedarf verzögern. Außerdem kann ein vorzeitiges Auslaugen von eventuellen Zusätzen verhindert werden. Als Vernetzungsmittel eignet sich beispielsweise Cyanamid, das Proteine und Polysaccharide vernetzt und beim biologischen Abbau keine körperfremden, schädlichen Reststoffe ergibt, da es zu Harnstoff degradiert wird.

Die erfindungsgemäße Kompositmatrix kann darüber hinaus biologisch aktive Verbindungen umfassen. Hierbei kann es sich beispielsweise um Verbindungen handeln, die die Eigenschaft der Matrix für die Besiedlung von Zellen optimieren, wie beispielsweise Antibiotika, Verbindungen zur Verbesserung der Zelladhäsion, Calciumsalze, induktive Faktoren oder weitere Glykosaminoglykane und deren Derivate. Vorteilhaft läßt sich die Zelladhäsion durch Zugabe von hochpolymerem Poly-L-lysin oder Beschichtung mit einem aktivierten Succinylderivat von Poly-L-lysin oder das Beimengen von Fibronektin oder Peptiden mit RGD-Sequenzen verbessern.

Um den Einsatz der erfindungsgemäßen Kompositmatrix in der Therapie von ossären Gewebedefekten zu optimierten, kann die Matrix Calciumsalze wie z.B. Calciumsufate, Calciumphosphate und Calciumcarbonate beispielsweise als Suspension oder Lösung enthalten.

Zur Verminderung der Infektionsgefahr bei der Implantation der erfindungsgemäßen Kompositmatrix kann diese auch Antibiotika enthalten.

Als weitere biologisch aktive Verbindungen kann die erfindungsgemäße Kompositmätrix beispielsweise zur Optimierung der Reparatur von muskuloskeletalen Defekten induktive Faktoren, insbesondere Cytokine wie z.B. bFGF (fibroblast growth factor), IGF (insulin-like growth factor) oder TGFbeta (transforming growth factor) enthalten.

Die Kompositmatrix eignet sich besonders für die in vitro und in vivo Generierung von differenziertem Gewebe aus chondrozytären Zellen, mesenchymalen Stamm- und Progenitorzellen, Osteoblasten und Bindegewebezellen, ausgenommen humane embryonale Zellen. Die Erfindung betrifft somit auch Kompositmatrizes, die diese Zellen umfassen.

Ein weiterer Aspekt der vorliegenden Erfindung ist ein Verfahren zur Herstellung der vorstehend beschriebenen porösen Kompositmatrix. Dieses Verfahren umfaßt das Lösen oder Suspendieren des Hyaluronsäurederivats und des hydrolysierten Kollagens in einem geeigneten ersten Lösungsmittel, die Zugabe einer pulverförmigen Verbindung, die sich in dem ersten Lösungsmittel praktisch nicht löst, die jedoch in einem zweiten Lösungsmittel löslich ist, in dem die Matrixbildner Hyaluronsäurederivat und hydrolysiertes Kollagen praktisch unlöslich sind, zu der Lösung oder Suspension, wobei die pulverförmige Verbindung eine mittlere Korngrößenverteilung im Bereich der gewünschten Porengröße der herzustellenden Kompositmatrix aufweist, das Entfernen des ersten Lösungsmittels und anschließend das Lösen der pulverförmigen Verbindung in einem zweiten Lösungsmittel, in dem sich die pulverförmige Verbindung löst und die Matrixbildner praktisch nicht lösen.

Als erstes Lösungsmittel eignet sich insbesondere 1,1,1,3,3,3-Hexafluorisopropanol (HFIP). Hierbei handelt es sich um eine hochflüchtige Flüssigkeit, in der sich veresterte Hyaluronsäure, hydrolysiertes Kollagen (Gelatine) sowie weitere, für die spezifischen Erfordernisse notwendige Substanzen wie z.B. Wachstumsfaktoren und Caiciumverbindungen gleichzeitig bei Raumtemperatur lösen bzw. suspendieren. Je größer die Molekülaggregate des hydrolysierten Kollagens sind, desto schlechter ist deren Löslichkeit in HFIP. Fibrilläres Kollagen wird nicht mehr gelöst.

Die Konzentrationen der Ausgangsstoffe in dem ersten Lösungsmittel sind für das erfindungsgemäße Verfahren unwesentlich und können variiert werden, solange handhabbare Lösungen bzw. Suspensionen erhalten werden. Dies betrifft sowohl die Konzentrationen der einzelnen Komponenten, als auch die Gesamtkonzentration der Komponenten in dem ersten Lösungsmittel. Die Einzelkonzentrationen von dem Hyaluronsäurederivat und dem hydrolysierten Kollagen bestimmen das Gewichtsverhältnis der beiden Komponenten im Endprodukt. Da zum Schluß das erste Lösungsmittel dem Endprodukt entzogen wird, bestimmt die Gesamtkonzentration die Dichte und Festigkeit des Endproduktes.

Für die Handhabung der erfindungsgemäßen Kompositmatrix spielt deren mechanische Festigkeit in nassem Zustand eine wichtige Rolle. Die Festigkeit der Kompositmatrix ist bei hohem Hyaluronsäurederivatanteil in der Matrix am größten, da die Matrix hier am wenigsten quillt. Bei wachsendem Anteil des hydrolysierten Kollagens quillt die Kompositmatrix zunehmend stark und wird weniger stabil.

Bevorzugt wird in dem erfindungsgemäßen Verfahren HYAFF mit 5 Gew.-% in HFIP gelöst. Hierzu kann Gelatine in variabler Einzelkonzentration von beispielsweise bis zu 7,5 Gew.-% beigegeben werden, so daß eine hohe Gesamtkonzentration von 12,5 Gew.-% Matrixbildner in der Lösung erreicht wird. Dadurch wird die Minderung der Festigkeit wegen der starken Quellung kompensiert. Lösungen mit einer Gesamtkonzentration von mehr als 12,5 Gew.-% sind sehr zäh und lassen sich schlecht handhaben. Eine Erhöhung des Anteils der Gelatine im Endprodukt ist daher nur dann durchführbar, wenn der HYAFF-Anteil reduziert wird. Jedoch mindert eine Reduktion des HYAFF-Anteils (beispielsweise auf eine Einzelkonzentration von 2,77 Gew.-% bei einer Gesamtkonzentration von 9,23 Gew.-% der Matrixbildner in der Lösung, was einem Gewichtsverhältnis von HYAFF zu Gelatine von 30:70 entspricht) die Stabilität der Matrix ohne eine Verbesserung der Bioverträglichkeit zu ergeben.

Grundsätzlich wirkt sich der Zusatz von Gelatine positiv auf die Bioverträglichkeit und auf die histogene Eigenschaft der Matrix aus. In vitro und in vivo bewährte sich ein Gewichtsverhältnis von Hyaluronsäurederivat zu hydrolysiertem Kollagen von etwa 70:30, wobei jedoch je nach Anwendung zum Beispiel für die Bildung von Knorpel oder für die Regeneration von Knochengewebe optimale Gewichtsverhältnisse im Bereich von 99:1 bis 60:40 liegen können.

Die Porenbildung erfolgt durch Zugabe einer pulverförmigen Verbindung, die in dem ersten Lösungsmittel praktisch nicht löslich ist. Wird als erstes Lösungsmittel HFIP verwendet, so eignete sich beispielsweise Natriumchlorid als pulverförmige Verbindung zur Porenbildung. Natriumchlorid ist praktisch unlöslich in HFIP, außerdem ist es untoxisch und billig.

Neben Natriumchlorid eignet sich darüber hinaus z.B. beim Einsatz von HFIP als erstes Lösungsmittel jedes wasserlösliche und in HFIP nicht lösliche Alkali- oder Erdalkalisalz, insbesondere -halogenid. Aus den oben genannten Gründen wird jedoch Natriumchlorid bevorzugt.

Die zugegebene Menge der pulverförmigen Verbindung bestimmt die Porenzahl und damit die Dichte und auch Festigkeit der hergestellten Matrix. Als vorteilhaft hat sich ein Gewichtsverhältnis von Lösung oder Suspension zu Natriumchloridkristallen von etwa 1:2 erwiesen.

Die Porengröße wird durch die Auswahl der Korngröße der pulverförmigen Verbindung bestimmt. Käufliches Natriumchlorid hat überwiegend Körner mit einem Durchmesser zwischen 500 und 1000 µm. Fraktionen von kleineren Größen können einfach durch zermörsern von größeren Körnern und durch Sieben durch kalibrierte Siebe hergestellt werden.

Das Gemisch aus HYAFF, Gelatine und Natriumchloridkristallen hat die Konsistenz einer dicken Paste. Durch das Pressen mit einem Stempel in Formen, beispielsweise aus innertem Kunststoff (PTFE, PE, PVC) ist es möglich. Matrixobjekte herzustellen, deren Form weitgehend dem Bedarf angepaßt werden kann. Da HFIP als Lösungsmittel sehr volatil ist, erfolgt ein schnelles Trocknen des Gemisches. Deswegen sollte das Gemisch in geschlossenen Gefäßen aufbewahrt und möglichst schnell verarbeitet werden. Das Trocknen kann beispielsweise über Nacht unter einem Abzug und anschließend einige Stunden im Vakuum erfolgen. Danach kann die Kompositmatrix aus der Form entnommen werden.

Da das Gemisch während der Trocknung kaum schrumpft, kann das Loslösen der Matrix Schwierigkeiten bereiten. Deswegen sollten die Formen so gestaltet sein, daß man den getrockneten Inhalt mit einem Stempel herausdrücken kann. Beispielsweise können zylinderförmige Matrizes mit einem Durchmesser von 3-18 mm und einer Höhe von 2-15 mm leicht hergestellt werden. Für die Herstellung von größeren Matrixblöcken haben sich kuboide, wannenförmige Formen als besonders geeignet erwiesen, die aus zerlegbaren Wand- und Bodenteilen zusammengelegt sind.

Die Poren der erfindungsgemäßen Kompositmatrix werden anschließend durch Lösen der pulverförmigen Verbindung in einem zweiten Lösungsmittel erhalten, in dem sich die pulverförmige Verbindung löst und die Matrixbildner praktisch nicht lösen. Wenn Natriumchloridkristalle als pulverförmige Verbindung verwendet werden, eignet sich als zweites Lösungsmittel insbesondere Wasser. Mehrfaches Spülen in Reinwasser entfernt das Salz und eventuell noch anhaftende Spuren von HFIP. Bei kleinen Proben empfehlen sich vier Wechsel des zweiten Lösungsmittels nach jeweils 15 Minuten Eintauchzeit, bei größeren Proben 6 Wechsel nach jeweils 20 Minuten.

Beim ersten Trocknen der Kompositmatrix durch Verdampfen des ersten Lösungsmittels werden primär geschlossene Poren mit darin enthaltenen Salzkörnern erzeugt. Während des Waschvorgangs quillt die semipermeable Substanz der Matrix auf und es kommt in den Poren zur Bildung einer osmotisch hochaktiven Salzlösung. Infolgedessen platzen bei weiterer Wasseraufnahme die Poren und die Matrix wird zum Schluß offenporig.

Falls gewünscht, kann die Kompositmatrix während oder nach der Herstellung zusätzlich mit wie oben beispielhaft genannten biologisch aktiven Verbindungen beladen werden. Vorteilhaft werden die biologisch aktiven Verbindungen zu der Lösung oder Suspension der Matrixkomponenten noch vor der Zugabe der pulverförmigen Verbindung zugesetzt.

Vorteilhaft wird die so hergestellte Kompositmatrix anschließend getrocknet. Dies kann beispielsweise durch Eintauchen in Aceton aufsteigender Konzentration (50%, 80%, 100%), blotten auf Filterpapier und anschließendes Trocknen im Vakuum erreicht werden.

Schließlich ist es ratsam, eine dünne Oberflächenschicht der Kompositmatrix beispielsweise mit einer scharfen Klinge zu entfernen, da in dieser Schicht eine hohe Zahl von geschlossenen Poren verbleibt, was das Eindringen von Zellen in die Tiefe behindert.

Vor einem Beladen mit Zellen wird die Kompositmatrix vorteilhaft sterilisiert. Dies kann durch verschiedene bekannte Sterilisationsverfahren wie beispielsweise mit Alkohol, Ethylenoxid oder durch gamma-Sterilisation erfolgen. Bevorzugt wird eine gamma-Sterilisation mit beispielsweise 350.000 rad.

Die erfindungsgemäße Kompositmatrix eignet sich für die in vitro und in vivo Generierung von differenziertem Gewebe aus chondrozytären Zellen, mesenchymalen Stamm- und Progenitorzellen, Osteoblasten und Bindegewebezellen ausgenommen humane embryonale Zellen. Durch eine spezifisch angepaßte Matrixzusammensetzung sowie Matrixgeometrie wird hiermit die Reparatur muskuloskeletaler Defekte möglich.

Die vorliegende Erfindung betrifft somit auch die Verwendung der oben beschriebenen Kompositmatrix zur in vitro Generierung von differenziertem Gewebe aus chondrozytären Zellen oder mesenchymalen Stamm- und Progenitorzellen ausgenommen humane embryonale Zellen, wobei frisch entnommene oder amplifizierte Zellen zu der Kompositmatrix zugegeben und unter chondro-, osteo- oder fibrogenen Bedingungen kultiviert werden.

Durch Zugabe von frisch entnommenen Chondrozyten oder mesenchymalen Stamm- und Progenitorzellen oder von in vitro amplifixierten, dedifferenzierten Chondrozyten oder mesenchymalen Stamm- und Progenitorzellen, ausgenommen humane embryonale Zellen, zu der erfindungsgemäßen Kompositmatrix unter chondrogenen Kulturbedingungen kann somit ein biomechanisch belastbares Gelenkknorpelgewebe hergestellt werden. Die erfindungsgemäße Kompositmatrix eignet sich somit zum Tissue Engineering von Gewebetypen des Binde- und Stützapparates, insbesondere von chondralem und ossärem Gewebe.

Eine so hergestellte, biokompatible und biodegradable Kompositmatrix eignet sich ohne oder mit vorheriger in vitro Kultivierung zur in vivo Differenzierung zu Gewebetypen des Binde- und Stützapparates, insbesondere zu chondralem und ossärem Gewebe unter ektoper oder autotoper Implantation.

Die erfindungsgemäße Kompositmatrix eignet sich beispielsweise für humane Chondrozyten, die aus hyalinem Knorpel gewonnen wurden. Dabei können hyaline Gelenkknorpel von Autopsien sowie Restknorpelbestände aus der Durchführung von , Totalendoprothesen den Entnahmeursprung darstellen.

Darüber hinaus können adulte mesenchymale Stamm- und Progenitorzellen beispielsweise aus Knochenmark, Synovium oder Periost sowie bevorzugt embryonale mesenchymale Stamm- und Progenitorzellen aus der Nabelschnur verwendet werden. Embryonale mesenchymale Stammzellen bieten den Vorteil einer fehlenden Abstoßung bei allogener Transplantation. Zudem stehen sie ständig in ausreichender Zahl zur Verfügung und können ohne einen zusätzlichen Eingriff am Patienten gewonnen werden.

Ein weiterer Aspekt der vorliegenden Erfindung ist ein Implantat, das eine erfindungsgemäße poröse Kompositmatrix umfaßt. Ein solches Implantat, das vorzugsweise mit der Kompositmatrix beschichtet ist, hat den Vorteil, daß eine bessere Integration zum Knochen und gegebenenfalls auch zum umgebenden Bindegewebe hergestellt wird.

Als Implantatoberflächen, die mit der erfindungsgemäßen Kompositmatrix beschichtet werden, eignen sich insbesondere Oberflächen aus Metall, wie beispielsweise Titan oder Stahl, einem Polymer oder Keramik.

Die erfindungsgemäße Kompositmatrix weist den Vorteil auf, daß es beim Beladen der Matrix mit Zellen nur zu einer geringen Größenveränderung kommt. Bekannte Kollagenmatrizes zeigen beim Beladen mit Zellen starke Größenunterschiede (zunächst massives Aufquellen, dann starke Tendenz sich einzukugeln). Für die Anwendung von Tissue-Engineering (in vitro Erzeugung von Gewebe mit dem Ziel, dieses in einen Defekt einzubauen) ist jedoch eine Größenstabilität der Matrix auch nach Zellbeladung und Kultivierung von großem Vorteil. Dies wird durch die erfindungsgemäße Kompositmatrix und insbesondere durch die gleichzeitige Mischung der Matrixbildner unter Verwendung von HFIP erreicht.

Darüber hinaus werden mit der erfindungsgemäßen Kompositmatrix folgende weitere Vorteile erzielt:

In stationärer Kultur ist eine Redifferenzierung amplifizierter Chondrozyten in der Kompositmatrix möglich. Es werden knorpeltypische Proteoglykane (Chondroitinsulfat, Keratansulfat, Aggregan) sowie Kollagen II gebildet. Dies konnte nach 2-, 4- und 6-wöchiger Kultivierung nachgewiesen werden.

Das in vitro erzeugte Produkt aus auf der Kompositmatrix kultivierten Chondrozyten zeigt eine deutliche Zunahme in der biomechanischen Stabilität im Vergleich zur Ausgangsbedingung am Zeitpunkt des Aufbringens von Zellen auf der Matrix.

Ohne Zellen löst sich die Matrix in vitro nach ca. 14 Tagen auf. In vivo läßt sich eine Resorption nach ca. 6-8 Wochen beobachten (Nacktmaus, Kaninchen).

Gute Differenzierungsfähigkeit von Knochenmarkzellen zu hyalinartigem Gewebe in vitro.

Sehr gute Differenzierung zu ossärem Gewebe beim Einbau in Subkutangewebe von Nacktmäusen, Resorption der Matrix in vivo erst nach 6 Wochen.

Beim Einbau in osteochondrale Defekte im Kniegelenk von Kaninchen ist eine Integration des Zell-Matrixkonstruktes erkennbar (Zellen waren hierbei Knochenmarkzellen oder Chondrozyten), Differenzierung zu Knorpelgewebe ist erkennbar. Im ossären Defektanteil zeigt sich knöcherne Integration und Ausdifferenzierung zu neuem Knochengewebe.

Keine Entzündungsneigung durch Material im Kniegelenk nachweisbar (kein Gelenkerguß, kein massiver Anstieg von Entzündungszellen).

Einbau des Zell-Matrixkonstruktes in Meniskusdefekte des Kaninchens nach vorheriger Kultivierung in vitro möglich, gute Regeneration des Meniskusdefektes, keine Entzündungsneigung im Kniegelenk.

In der Kombination mit dem Hyaluronsäurederivat ist das sonst auftretende negative Quellen bei Gelatine oder Kollagen nach Befeuchten der trockenen Matrix nicht gegeben. Dies ist für Tissue Engineering Matrizes, bei denen ein bestimmtes vorgegebenes Defektareal zu reparieren ist, besonders günstig.

Die anliegenden Figuren zeigen Vergrößerungeri der erfindungsgemäßen Kompositmatrix. Es zeigen:
Figur 1 eine 50-fache Vergrößerung einer stabileren Struktur (oben) und einer schwächeren Struktur (unten),
Figur 2 eine 100-fache Vergrößerung der stabileren Struktur (oben) und der schwächeren Struktur (unten),
Figur 3 eine 200-fache Vergrößerung der stabileren Struktur (oben) und der schwächeren Struktur (unten) und
Figur 4 in den Poren wachsende Chondrozyten bei 200-facher Vergrößerung (oben) und 1000-facher Vergrößerung (unten).

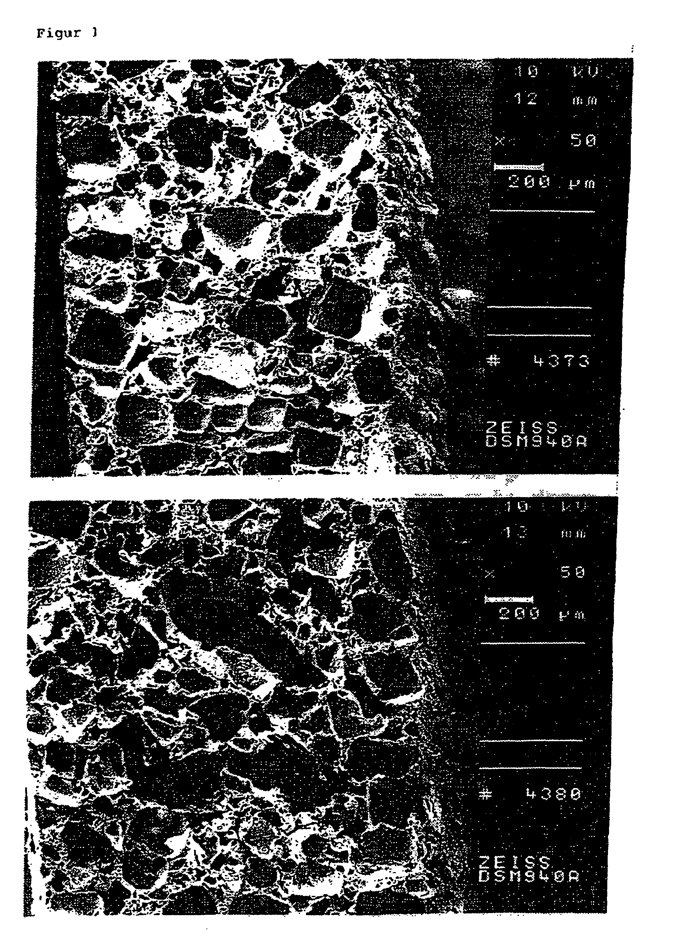

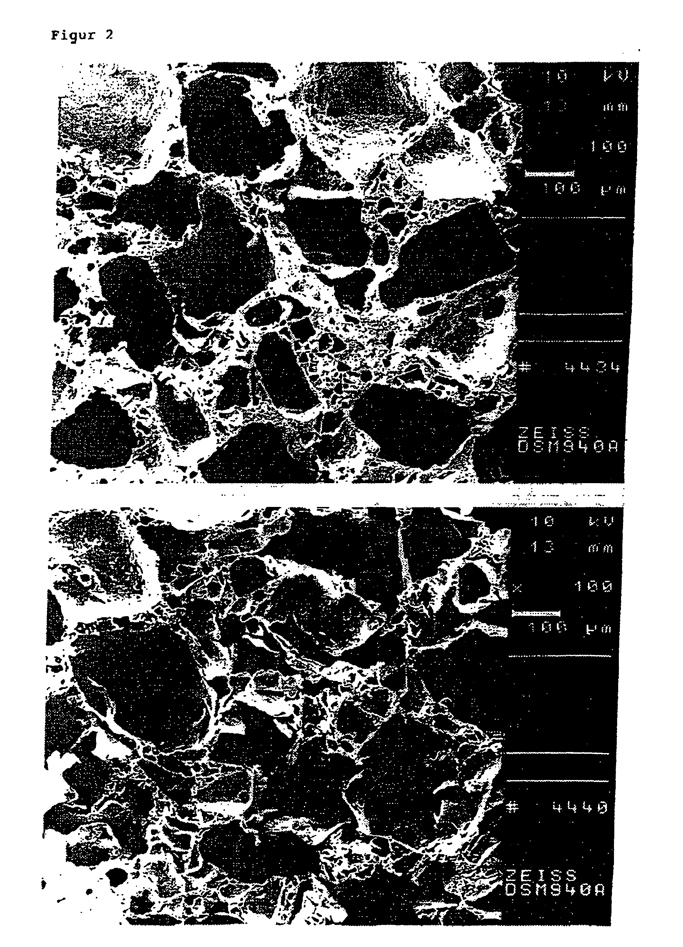

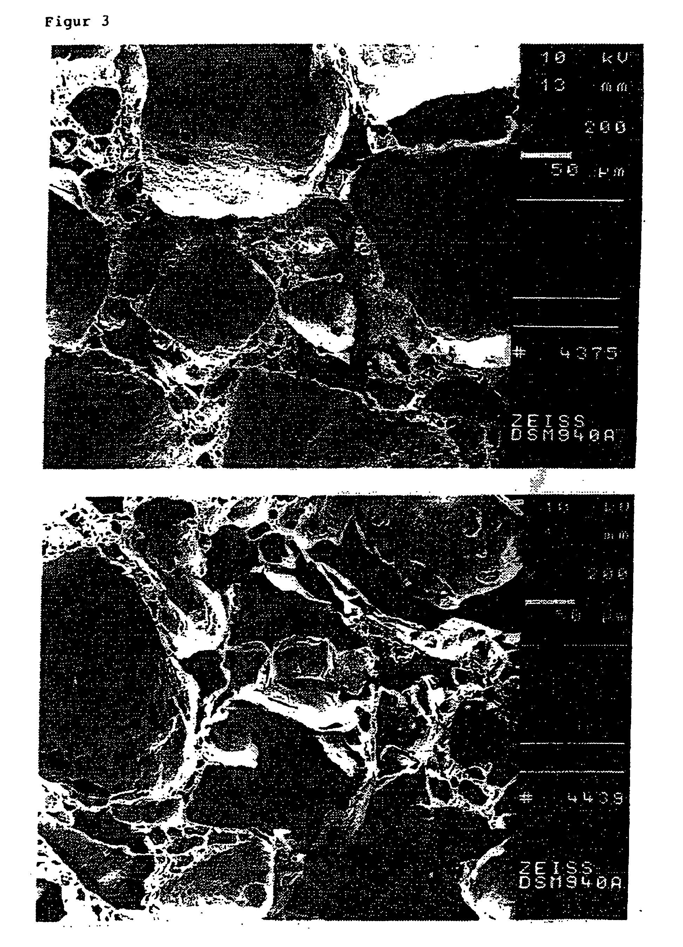

Die jeweils stabilere Struktur in Figuren 1-3 wurde durch eine höhere Gesamtkonzentration der Matrixkomponenten in der Lösung während der Herstellung erhalten, wobei die Gesamtkonzentration von 6,3% für die schwächere Struktur bis 9,2% für die stabilere Struktur variiert wurde.

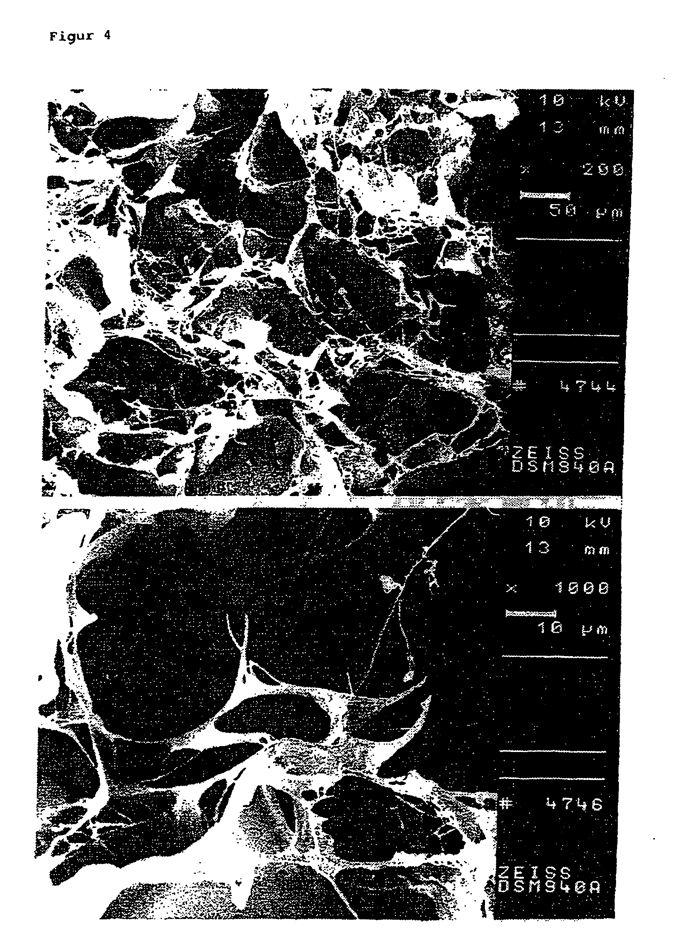

Die fadenartigen Strukturen in Figur 4 deuten auf die beginnende Produktion der extrazellulären Matrix hin.

Die nachfolgenden Beispiele sollen die vorliegende Erfindung näher erläutern.

### Beispiel 1

Eine erfindungsgemäße Kompositmatrix wurde durch Auflösen von HYAFF und Gelatine in HFIP, Zugabe von Natriumchloridkristallen, Formen und Trocknen der entstandenen Paste, sowie durch Entfernen des Natriumchlorids durch mehrfaches Spülen mit Wasser hergestellt. Nach dem Trocknen wurde die Kompositmatrix einer gamma-Sterilisation unterzogen.

### Beispiel 2

### Präparationsbeschreibung

### Gewinnung von Chondrozyten für die primäre Zellkultur

### Adulter Gelenkknorpel

Hyaliner Gelenkknorpel (adult) wurde nach Entnahme sofort in RPMI-Medium transferiert und möglichst schnell aufgearbeitet (kleiner 24h). Hierzu wurde der Knorpel zunächst mechanisch vom Knochen getrennt und dann mit einem Skalpell zerkleinert (Endgröße: 1-2 mm große Knorpelstückchen). Bei 37°C erfolgte daraufhin unter ständigem Schwenken ein enzymatischer Andau mit Kollagenase, Hyaluronidase und DNAse. Die Enzyme wurden in RPMI-Medium mit Hepes-Puffer, L-Glutamin und Zusatz von Penicillin/Streptomycin (Antibiotikazusatz) resuspendiert. Das optimierte enzymatische Dissoziationsintervall betrug 12 Stunden. Durch Zugabe von serumhaltigem Medium (RPMI mit 10% AB-Serum oder RPMI mit 10% fetalem Rinderserum (FBS)) wurde die enzymatische Aktivität gepuffert. Daraufhin erfolgte die Extraktion noch verbliebener extrazellulärer Matrixstücke durch Filtern und Zentrifugieren der Suspension, Verwerfen des Überstandes und Resuspendieren des Zellpellets in RPMI mit 10% AB-Serum oder RPMI mit 10% FBS. Nach einer Zellzählung folgte eine stationäre Kultivierung und AmpiifLkation der Chondrozyten für 14-21 Tage (2 mal pro Woche Mediumwechsel mit serumhaltigem Medium). Nach Erreichen von Konfluenz erfolgte ein Passagieren der Zellen (Entfernen des Mediums, Zugabe von 0,25% Trypsin, nach Aufheben der Zelladhärenz Zugabe von serumhaltigem Medium, Zentrifugation der Zellsuspension und Resuspendieren des daraufhin gewonnenen Pellets in frischem Medium, Zellzählung und erneutes Aussähen der Zellen). Hierbei wurde zumeist eine 1 in 4 Teilung durchgeführt, d.h. eine Kulturflasche lieferte die Zellen für 4 neue Flaschen. Nach erneuter Konfluenz (ca. nach 2-4 Wochen) wurden die Zellen (Sekundärkultur) durch erneute Trypsinbehandlung (siehe oben) von dem Kulturboden abgelöst und für die Beladung der Matrizes vorbereitet.

Es können jedoch auch Zellen in Primär- und Tertiärkultur (2 Passagierungsschritte) verwendet werden.

### Embryonale mesenchymale Stamm- und Progenitorzellen aus der Nabelschnur

Die Zellen wurden in DMEM Medium mit 10% FBS kultiviert und nach Erreichen von Konfluenz als Primärkultur für die Matrixbeladung verwendet. Die Gewinnung der adhärenten Zellen erfolgte wiederum durch die oben beschriebene Trypsinanwendung.

### Adulte mesenchymale Stamm- und Progenitorzellen aus Knochenmark

Knochenmark wurde aus dem Darmbeinkamm von 4 Monate alten weißen Neuseelandhasen gewonnen. Zu dem Aspirat wurde "Dolbecco's modified Eagle's Medium" (DMEM) mit 10% fetalem Rinderserum (FBS) zugegeben. Nach Bestimmung der Zellzahl wurden 20x10⁶ Zellen in 100 mm Zuchtschalen bei 37°C mit 5% CO₂ kultiviert. Das Medium wurde zweimal pro Woche gewechselt bis die Zellen 80% konfluent waren. Adhärente Zellen wurde wie oben beschrieben mit Trypsin behandelt, gezählt, gewaschen und in DMEM auf eine Endkonzentration von 5x10⁵ Zellen/25 µL resuspendiert.

### Matrixbeladung

Zur Matrixbeladung wurde das Zellpellet in wenig Medium aufgenommen (1 mm3/1 µl). Daraufhin erfolgte das Beladen der Matrix (steril, bisher trocken) von einer Seite. Dadurch wurde das Entweichen von Luft, die sich in der Matrix befand, sichergestellt (Einwirkzeit 1-5 Minuten). Daraufhin wurde durch Erzeugen eines Unter- und Überdruckes mit einer Pipettenspitze noch verbliebenes Medium mit hoher Zellkonzentration in die Matrix transferiert. Es sollte eine möglichst homogene Verteilung von Zellen in der Matrix erreicht werden. Durch die Zugabe von Detergenzstoffen kann die Beladung erleichtert werden.

Anschließend erfolgte eine Inkubation von 2h im Inkubator (37°C, 5 % CO₂). Dies erlaubte den Zellen, sich an der vorliegenden Matrix zu adhärieren.

Abschließend wurde das Zell-Matrixkonstrukt mit Medium voll überschichtet und weiterkultiviert.

### Kulturbeschreibung

### Stationäre Kultur

Hierfür wurden unterschiedliche Kulturmedien verwendet:
RPMI mit 10% AB-Serum (Human),
RPMI mit 10% FBS oder

"Dulbecco's modified Eagle's Medium" (DMEM) mit hohem Glucosegehalt und Nähr- sowie Zusatzstoffen (ITS und Pyruvat plus Dexamethason, Ascorbinsäure und TGF betal). (Vgl. B. Johnstone in Exp. Cell Res. 238 (1998)).

### Kontinuierliche Perfusionskultur

Eine alternative Kultivierungsbedingung stellt die kontinuierliche Perfusionskultur in RPMI-Medium mit 10% AB-Serum dar.

### Beispiel 3

Um die Wirksamkeit der Beladung der erfindungsgemäßen Kompositmatrix mit Zellen zu untersuchen, wurde eine gemäß Beispiel 1 hergestellt Kompositmatrix mit den gemäß Beispiel 2 gewonnenen Knochenmarkzellen beladen. Ein Teil der Matrizes wurde sofort in Formalin fixiert, gewaschen und in Paraffin eingebettet (Gruppe I). Andere beladene Matrizes (Gruppe II) wurden für 14 Tage in einem chondrogenen Medium enthaltend DMEM mit ITS + Vormischung (Collaborative Biomechanical Products), Pyruvat (1mM), Ascorbinsäure-2-phosphat (37,5 µg/ml), Dexamethason (10⁻⁷M) und TGF-β1 (10 ng/ml), kultiviert. Das Medium wurde dreimal wöchentlich gewechselt.

Die Zellsuspension wurde unter leichtem Quellen des Konstrukts in die Matrix eingebracht. Durch Toluidinblau gefärbte Bereiche der Matrizes aus Gruppe I konnte gezeigt werden, daß eine hohe Zellbeladung in allen Poren der Matrizes vorlag. Nach 14 Tagen unter chondrogenen Kulturbedingungen (Gruppe II) waren die ursprünglich weichen Zellmatrixkonstrukte gehärtet. Die Tuluidinblau gefärbten Bereiche zeigten Zellen mit einer ausgeprägten metachromatisch färbenden extrazellulären Matrix, die in der gesamten Kompositmatrix vorlag. Die extrazelluläre Matrix enthielt Kollagen II.

### Beispiel 4

Wie in Beispiel 3 mit Zellen beladene Kompositmatrizes wurden subkutane in immundefiziente Mäuse implantiert (Gruppe III). Gleiche Matrizes wurden für 14 Tage in vitro in dem in Beispiel 3 beschriebenen chondrogenen Medium kultiviert und dann in vivo implantiert (Gruppe IV). Die Implantate wurden nach 3 Wochen entnommen, in Formalin fixiert, entkalkt und in Paraffin eingebettet.

Es wurden 5 µm dicke Scheiben der Proben geschnitten und mit Toluidinblau gefärbt. Die gefärbten Bereiche wurden nach ihrer osteochondralen Differenzierung bewertet (0 (weder Knochen noch Knorpel in den Matrixporen) bis 4 (mehr als 75% der Poren enthalten Knochen und/oder Knorpel)).

Nach 3 Wochen in vivo trat weder bei den Gruppe III noch bei den Gruppe IV Implantaten eine wesentliche Größenveränderung auf. Die für 14 Tage in vitro vorkultivierten Implantate (Gruppe IV) erschienen jedoch qualitativ härter als die Kompositmatrizes, die sofort nach der Beladung mit den Zellen implantiert wurden (Gruppe III). Das Färben mit Toluidinblau ergab eine osteochondrale Differenzierung von Zellen in den Kompositmatrizes beider Gruppen, wobei sich die Poren mit Knorpel und Knochen füllten. Die Kompositmatrizes der Gruppe IV enthielten jedoch mehr Knochen und Knorpel (durchschnittliche Bewertung = 4, verglichen mit einer Bewertung von 3 für Gruppe III). Außerdem war der Anteil an Knochen in den Proben der Gruppe IV größer (Verhältnis von Knochen: Knorpel: fibrösem Gewebe in Gruppe III: 40:20:40 und in Gruppe IV: 85:10:5).

## Patentansprüche

1. Poröse Kompositmatrix, wobei die Matrix aus Matrixbildnern umfassend ein Hyaluronsäurederivat und hydrolysiertes Kollagen aufgebaut ist, und die Matrixbildner in einem Gewichtsverhältnisbereich von Hyaluronsäurederivat zu hydrolysiertem Kollagen von 30:70 bis 99:1 vorliegen.

2. Kompositmatrix nach Anspruch 1, worin die Matrixbildner in einem Gewichtsverhältnisbereich von Hyaluronsäurederivat zu hydrolysiertem Kollagen von 60:40 bis 99:1, bevorzugt in einem Gewichtsverhältnis von etwa 70:30 vorliegen.

3. Kompositmatrix nach einem der vorhergehenden Ansprüche, worin das hydrolysierte Kollagen partiell und/oder vollständig hydrolysiert ist.

4. Kompositmatrix nach einem der vorhergehenden Ansprüche, worin das hydrolysierte Kollagen zusätzlich derivatisiert und/oder quervernetzt ist.

5. Kompositmatrix nach einem der vorhergehenden Ansprüche, worin das Hyaluronsäurederivat ein Hyaluronsäureester ist.

6. Kompositmatrix nach Anspruch 5, worin der Hyaluronsäureester ein Ethyl- oder Benzylester der Hyaluronsäure ist.

7. Kompositmatrix nach einem der vorhergehenden Ansprüche, umfassend Poren mit einem durchschnittlichen Durchmesser im Bereich von 10-1000 µm.

8. Kompositmatrix nach Anspruch 7, worin die Poren einen durchschnittlichen Durchmesser im Bereich von 100-350 µm aufweisen.

9. Kompositmatrix nach Anspruch 7, worin die Poren einen durchschnittlichen Durchmesser im Bereich von 350-1000 µm aufweisen.

10. Kompositmatrix nach Anspruch 8 oder 9, worin zusätzlich Poren im Bereich von 10-100 µm vorhanden sind.

11. Kompositmatrix nach einem der vorhergehenden Ansprüche, die Quervernetzungen aufweist.

12. Kompositmatrix nach einem der vorhergehenden Ansprüche, umfassend biologisch aktive Verbindungen wie Antibiotika, Verbindungen zur Verbesserung der Zelladhäsion, Calciumsalze, induktive Faktoren oder weitere Glykosaminoglykane und deren Derivate.

13. Kompositmatrix nach einem der vorhergehenden Ansprüche, umfassend Chondrozyten, mesenchymale Stamm- und Progenitorzellen, Osteoblasten oder Bindegewebezellen, ausgenonnen aus humanen Embryos gewonnene Zellen.

14. Verfahren zur Herstellung einer porösen Kompositmatrix gemäß einem der Ansprüche 1-13, umfassend das Lösen oder Suspendieren des Hyaluronsäurederivats und des hydrolysierten Kollagens in einem geeigneten ersten Lösungsmittel, die Zugabe einer pulverförmigen Verbindung, die sich in dem ersten Lösungsmittel praktisch nicht löst, die jedoch in einem zweiten Lösungsmittel löslich ist, in dem die Matrixbildner Hyaluronsäurederivat und hydrolysiertes Kollagen praktisch unlöslich sind, zu der Lösung oder Suspension, wobei die pulverförmige Verbindung eine mittlere Korngrößenverteilung im Bereich der gewünschten Porengröße der herzustellenden Kompositmatrix aufweist, das Entfernen des ersten Lösungsmittels und anschließend das Lösen der pulverförmigen Verbindung in einem zweiten Lösungsmittel, in dem sich die pulverförmige Verbindung löst und die Matrixbildner praktisch nicht lösen.

15. Verfahren nach Anspruch 14, worin das erste Lösungsmittel 1, 1,1,3,3,3-Hexafluorisopropanol ist.

16. Verfahren nach Anspruch 14 oder 15, worin die pulverförmige Verbindung ein wasserlösliches Alkali- oder Erdalkalisalz, insbesondere ein Alkalihalogenid wie Natriumchlorid ist.

17. Verfahren nach einem der Ansprüche 14-16, worin das zweite Lösungsmittel Wasser ist.

18. Verfahren nach einem der Ansprüche 14-17, worin die Kompositmatrix zusätzlich geformt, getrocknet und gegebenenfalls sterilisiert wird.

19. Verfahren nach einem der Ansprüche 14-18, worin die Kompositmatrix zusätzlich gegebenenfalls mit biologisch aktiven Verbindungen und Chondrozyten, mesenchymalen Stammund Progenitorzellen, Osteoblasten oder Bindegewebezellen, ausgenonnen aus humanen Embryos gewonnene Zellen beladen wird.

20. Verwendung einer Kompositmatrix nach einem der Ansprüche 1-13 zur in vitro Generierung von differenziertem Gewebe aus chondrozytären Zellen oder mesenchymalen Stamm- und Progenitorzellen ausgenommen aus humanen Embryos gewonnene Zellen, wobei frisch entnommene oder amplifizierte Zellen zu der Kompositmatrix zugegeben und unter chondro-, osteo- oder fibrogenen Bedingungen kultiviert werden.

21. Verwendung nach Anspruch 20 zum Tissue Engineering von Gewebstypen des Binde- und Stützapparates, insbesondere von chondralem und ossärem Gewebe.

22. Verwendung einer Kompositmatrix nach einem der Ansprüche 1-13 zur Herstellung eines Arzneimittels für die Reparatur muskuloskeletaler Defekte, wobei frisch entnommene oder amplifizierte chondrozytäre Zellen oder mesenchymale Stamm- und Progenitor zellen ausgenommen humane embryonale zellen zu der Kompositmatrix zugegeben und gegebenenfalls unter chondro-, osteo- oder fibrinogenen Bedingungen kultiviert werden und wobei die Zellen zu Gewebstypen des Binde- und Stützapparats, insbesondere zu chondralem und ossärem Gewebe differenzieren.

23. Implantat, umfassend eine poröse Kompositmatrix nach einem der Ansprüche 1-13.

24. Verfahren zur Herstellung eines Implantats gemäß Anspruch 23, worin eine poröse Kompositmatrix nach einem der Ansprüche 1-13 auf die Implantatoberfläche aufgeschichtet wird.

## Claims

1. A porous composite matrix, in which the matrix is constructed from matrix formers comprising a hyaluronic acid derivative and a hydrolyzed collagen, and the matrix formers are present in a weight ratio range of hyaluronic acid derivative to hydrolyzed collagen of 30:70 to 99:1.

2. The composite matrix as claimed in claim 1, in which the matrix formers are present in a weight ratio range of hyaluronic acid derivative to hydrolyzed collagen of 60:40 to 99:1, preferably in a weight ratio of approximately 70:30.

3. A composite matrix as claimed in one of the preceding claims, in which the hydrolyzed collagen is partially and/or completely hydrolyzed.

4. A composite matrix as claimed in one of the preceding claims, in which the hydrolyzed collagen is additionally derivatized and/or crosslinked.

5. A composite matrix as claimed in one of the preceding claims, in which the hyaluronic acid derivative is a hyaluronic acid ester.

6. A composite matrix as claimed in claim 5, in which the hyaluronic acid ester is an ethyl or benzyl ester of hyaluronic acid.

7. A composite matrix as claimed in one of the preceding claims, comprising pores having an average diameter in the range of 10-1000 µm.

8. A composite matrix as claimed in claim 7, in which the pores have an average diameter in the range of 100-350 µm.

9. A composite matrix as claimed in claim 7, in which the pores have an average diameter in the range of 350-1000 µm.

10. A composite matrix as claimed in claim 8 or 9, in which pores in the range of 10-100 µm are additionally present.

11. A composite matrix as claimed in one of the preceding claims, which has crosslinkages.

12. A composite matrix as claimed in one of the preceding claims, comprising biologically active compounds such as antibiotics, compounds for improving cell adhesion, calcium salts, inductive factors or further glycosaminoglycans and their derivatives.

13. A composite matrix as claimed in one of the preceding claims, comprising chondrocytes, mesenchymal stem and progenitor cells, osteoblasts or connective tissue cells, except cells obtained from human embryos.

14. A process for the production of a porous composite matrix as claimed in one of claims 1-13, comprising the dissolution or suspension of the hyaluronic acid derivative and the hydrolyzed collagen in a suitable first solvent, the addition of a pulverulent compound which is virtually insoluble in the first solvent, but which is soluble in a second solvent, in which the matrix formers hyaluronic acid derivative and hydrolyzed collagen are virtually insoluble, to the solution or suspension, the pulverulent compound having an average particle size distribution in the range of the desired pore size of the composite matrix to be produced, the removal of the first solvent and subsequently the dissolution of the pulverulent compound in a second solvent, in which the pulverulent compound dissolves and the matrix formers are virtually insoluble.

15. The process as claimed in claim 14, in which the first solvent is 1,1,1,3,3,3-hexafluoroisopropanol.

16. The process as claimed in claim 14 or 15, in which the pulverulent compound is a water-soluble alkali metal or alkaline earth metal salt, in particular an alkali metal halide such as sodium chloride.

17. The process as claimed in one of claims 14-16, in which the second solvent is water.

18. The process as claimed in one of claims 14-17, in which the composite matrix is additionally shaped, dried and optionally sterilized.

19. The process as claimed in one of claims 14-18, in which the composite matrix is additionally optionally loaded with biologically active compounds and chondrocytes, mesenchymal stem and progenitor cells, osteoblasts or connective tissue cells, except cells obtained from human embryos.

20. The use of a composite matrix as claimed in one of claims 1-13 for the in vitro generation of differentiated tissue from chondrocytic cells or mesenchymal stem and progenitor cells, except cells obtained from human embryos, freshly removed or amplified cells being added to the composite matrix and cultured under chondro-, osteo- or fibrogenic conditions.

21. The use as claimed in claim 20 for the tissue engineering of tissue types of the connective and supportive apparatus, in particular of chondral and osseous tissue.

22. The use of a composite matrix as claimed in one of claims 1-13 for the production of a pharmaceutical agent for repairing musculoskeletal defects, wherein freshly removed or amplified chondrocytic cells or mesenchymal stem and progenitor cells, except human embryonic cells, are added to the composite matrix and are optionally cultured under chondro-, osteo- or fribrogenic conditions and wherein the cells differentiate to give tissue types of the connective and supportive apparatus, in particular of chondral and osseous tissue.

23. An implant, comprising a porous composite matrix as claimed in one of claims 1-13.

24. A process for the production of an implant as claimed in claim 23, in which a porous composite matrix as claimed in one of claims 1-13 is coated onto the implant surface.

## Revendications

1. Matrice composite poreuse, dans laquelle la matrice est constituée d'agents générateurs de matrice comportant un dérivé d'acide hyaluronique et du collagène hydrolysé, et dans laquelle les agents générateurs de matrice se présentent dans une gamme de rapports pondéraux entre le dérivé d'acide hyaluronique et le collagène hydrolysé de 30/70 à 99/1.

2. Matrice composite suivant la revendication 1, dans laquelle les agents générateurs de matrice se présentent dans une gamme de rapports pondéraux entre le dérivé d'acide hyaluronique et le collagène hydrolysé de 60/40 à 99/1, de préférence dans un rapport pondéral d'environ 70/30.

3. Matrice composite suivant l'une des revendications précédentes, dans laquelle le collagène hydrolysé est hydrolysé partiellement et/ou totalement.

4. Matrice composite suivant l'une des revendications précédentes, dans laquelle le collagène hydrolysé est en supplément transformé en dérivé et/ou réticulé transversalement.

5. Matrice composite suivant l'une des revendications précédentes, dans laquelle le dérivé d'acide hyaluronique est un ester d'acide hyaluronique.

6. Matrice composite suivant la revendication 5, dans laquelle l'ester d'acide hyaluronique est un ester éthylique ou benzylique de l'acide hyaluronique.

7. Matrice composite suivant l'une des revendications précédentes, comportant des pores ayant un diamètre moyen de l'ordre de 10-1000 µm.

8. Matrice composite suivant la revendication 7, dans laquelle les pores présentent un diamètre moyen de l'ordre de 100-350 µm.

9. Matrice composite suivant la revendication 7, dans laquelle les pores présentent un diamètre moyen de l'ordre de 250-1000 µm.

10. Matrice composite suivant l'une des revendications 8 et 9, dans laquelle il y a en supplément des pores dans la gamme de 10-100 µm.

11. Matrice composite suivant l'une des revendications précédentes, qui présente des réticulations transversales.

12. Matrice composite suivant l'une des revendications précédentes, comportant des composés biologiquement actifs, tels que des antibiotiques, des composés destinés à améliorer l'adhésion cellulaire, des sels de calcium, des facteurs inductifs ou d'autres glycosaminoglycanes et leurs dérivés.

13. Matrice composite suivant l'une des revendications précédentes, comportant des chondrocytes, des cellules de souche et progénitrices de mésenchyme, des ostéoblastes ou des cellules de tissu conjonctif, à l'exclusion des cellules obtenues à partir d'embryons humains.

14. Procédé de préparation d'une matrice composite poreuse suivant l'une des revendications 1 à 13, comprenant la solution ou mise en suspension du dérivé d'acide hyaluronique et du collagène hydrolysé dans un premier solvant approprié, l'addition d'un composé pulvérulent, qui ne se dissout pratiquement pas dans le premier solvant, mais qui est soluble dans un deuxième solvant dans lequel les agents générateurs de matrice, le dérivé d'acide hyaluronique et le collagène hydrolysé, sont pratiquement insolubles, à la solution ou suspension, le composé pulvérulent présentant une répartition de tailles granulaires moyenne dans la gamme des grandeurs de pores souhaitées de la matrice composite à préparer, l'élimination du premier solvant et ensuite la dissolution du composé pulvérulent dans un deuxième solvant, dans lequel le composé pulvérulent se dissout et les agents générateurs de matrice ne se dissolvent pratiquement pas.

15. Procédé suivant la revendication 14, dans laquelle le premier solvant est du 1,1,1,3,3,3 hexafluorisopropanol.

16. Procédé suivant l'une des revendications 14 et 15, dans lequel le composé pulvérulent est un sel de ,étal alcalin ou alcalino-terreux soluble dans l'eau, en particulier un halogénure de métal alcalin, tel que du chlorure de sodium.

17. Procédé suivant l'une des revendications 14 à 16, dans lequel le deuxième solvant est de l'eau.

18. Procédé suivant l'une des revendications 14 à 17, dans lequel la matrice composite est en supplément moulée, séchée et éventuellement stérilisée.

19. Procédé suivant l'une des revendications 14 à 18, dans lequel la matrice composite est en supplément éventuellement chargée de composés biologiquement actifs et de chondrocytes, de cellules de souche et progénitrices de mésenchyme, d'osteoblastes ou de cellules de tissu conjontif, à l'exception de cellules obtenues à partir d'embryons humains.

20. Utilisation d'une matrice composite suivant l'une des revendications 1 à 13, pour la formation in vitro d'un tissu différencié à partir de cellules chondrocytaires ou de cellules de souche et progénitrices de mésenchyme, à l'exception de cellules obtenues à partir de d'embryons humains, des cellules fraîchement prélevées ou amplifiées étant ajoutées à la matrice composite et étant cultivées dans des conditions chondrogènes, ostéogènes ou fibrogènes.

21. Utilisation suivant la revendication 20, pour l'ingénierie tissulaire de types de tissu de l'appareil conjonctif et de soutien, en particulier de tissu chondrique et osseux.

22. Utilisation d'une matrice composite suivant l'une des revendications 1 à 13, pour la fabrication d'un médicament destiné à la réparation de défauts musculo-squelettiques, dans laquelle des cellules chondrocytaires ou des cellules de souche et progénitrices de mésenchyme fraîchement prélevées ou amplifiées, à l'exception de cellules embryonnaires humaines, sont ajoutées à la matrice composite et sont éventuellement cultivées dans des conditions chondrogènes, ostéogènes ou fibrogènes, et dans laquelle les cellules se différencient en types tissulaires de l'appareil conjonctif et de soutien, en particulier en tissu chondrique et osseux.

23. Implant, comportant une matière composite poreuse suivant l'une des revendications 1 à 13.

24. Procédé de préparation d'un implant suivant la revendication 23, dans lequel une matrice composite poreuse suivant l'une des revendications 1 à 13 est appliquée sur la surface de l'implant.
